# EUROPEAN PATENT APPLICATION

(11) **EP 1 437 095 A1**
(43) Date of publication of application: **14.07.2004**
(21) Application number: 02800269.9
(22) Date of filing: 27.09.2002
(51) Int. Cl.: A61B 8/06

(54) **ULTRASONIC DIAGNOSING DEVICE AND ULTRSONIC DIAGNOSING METHOD**

(30) Priority: 27.09.2001 JP 2001297613
(71) Applicant: Hitachi Medical Corporation, Tokyo 100-0047 (JP)
(72) Inventor: MATSUMURA, Takeshi, Kashiwa-shi, Chiba 277-0825 (JP)
(74) Representative: Strehl Schübel-Hopf & Partner
(86) International application number: PCT/JP2002/010052
(87) International publication number: WO 2003/028556

(57) **Abstract**

An ultrasonic diagnosing device which irradiates and scans an object to be examined with ultrasound beams produced by driving a plurality of vibrators (23), and produces and displays the tomographic image of the object based on an ultrasonic wave reflected off this object, wherein a sound pressure regulating means for regulating the sound pressure of ultrasound beams during ultrasound beam scanning is provided so as to be able to make equal the sound pressures of ultrasound beams (53, 55, 57) in respective regions of interest (47, 49, 51). Therefore, the sound pressures of ultrasound beams (53, 55, 57) applied to contrast media in respective regions of interest (47, 49, 51) are made constant even when the regions (47, 49, 51) are at different depths to allow contrast media to give the same behavior, whereby uniform contrast effects are provided in the regions of interest (47, 49, 51). 0

## Description

### TECHNICAL FIELD

The present invention relates to an ultrasound imaging apparatus, more particularly to an ultrasound diagnostic apparatus suitable for conducting diagnosis by injecting a contrast medium into a blood vessel or the like of an object to be examined.

### BACKGROUND OF THE INVENTION

An ultrasound diagnostic apparatus transmits and receives ultrasonic waves between a probe and an object to be examined and generates a tomogram or the like on the basis of received signals, i.e., reflected echo signals, including waves reflected from the object, for providing information useful for diagnosis.

Generally, the probe has a plurality of transducers arranged like a straight line, like a curved line, or like a plane. A plurality of selected transducers are driven at the same time to form ultrasound beams, a region under diagnosis in the object is scanned, and a tomogram is generated on the basis of reflected echo signals made of reflected waves. Usually, the ultrasound beams emitted from the probe are subjected to focusing. The focusing is, for example, a known electronic focusing method for uniforming wave fronts of ultrasonic waves radiated from the respective transducers at an arbitrary focal point.

Meanwhile, ultrasound angiography for enhancing a contrast of bloodstream by injecting a contrast medium into a blood vessel has been proposed in taking a tomogram with an ultrasound diagnostic apparatus. The contrast medium is, for example, a known one which forms bubbles in the blood vessel. By detecting change of acoustic property of echoes generated when the bubbles are burst or vibrated, the contrast can be enhanced. That is, by the contrast effects brought about by behavior of the contrast medium, the tomogram of a region of interest is made clear.

Conventionally, because ultrasonic waves are attenuated in correspondence with the depth when ultrasonic waves having an identical strength are radiated from the respective transducers for scanning inside the object, when regions of interest exist in both of a shallow portion and a deep portion in the object, the contrast effect of the contrast medium at each of the depths in the region of interest varies. As a result, the contrast degree of the contrast medium with respect to the depths of the regions of interest become non-uniform to thereby cause an occasion where diagnosis is not proper. In Japanese Unexamined Patent Publication No. Hei. 11-155858, an ultrasound apparatus for adaptively controlling a transmission sound pressure so as to maximize a received signal from the contrast medium is disclosed. However, even if adapted transmitted beams are applied to a plurality of portions to enhance the received signal from the contrast medium, the effects of the contrast medium in a deep region of interest and in a shallow region scatter.

### SUMMARY OF THE INVENTION

To solve the above-stated problems, an ultrasound diagnostic apparatus, in which ultrasound beams generated by driving a probe are radiated to scan an object to be examined and a tomogram of the object is generated and displayed on the basis of ultrasonic waves reflected from the object, includes sound pressure adjusting means for adjusting the sound pressures of the ultrasound beams. Accordingly, the sound pressures of ultrasound beams in regions of interest can be made uniform. Therefore, even when regions of interest exist in various depths, the sound pressures of ultrasound beams radiated to the contrast medium in the respective regions of interest are made constant and the contrast mediums show similar behavior, whereby the degree of contrast effect in a plurality of regions of interest can be uniformed.

An object of the present invention is to make uniform the degree of contrast effect in a plurality of regions of interest (ROI) in an ultrasound tomogram.

### BRIEF DESCRIPTION OF THE DRAWINGS

Fig.1 is a schematic diagram showing one embodiment of an ultrasound diagnostic apparatus to which the present invention is applied;
Fig.2 is a flow chart showing a part of diagnostic procedure using the ultrasound diagnostic apparatus shown in Fig.1; and
Fig.3 is a diagram schematically illustrating a probe 1 shown in Fig.1 and an object to be examined to which ultrasound beams are radiated.

### BEST MODE FOR CARRYING OUT THE INVENTION

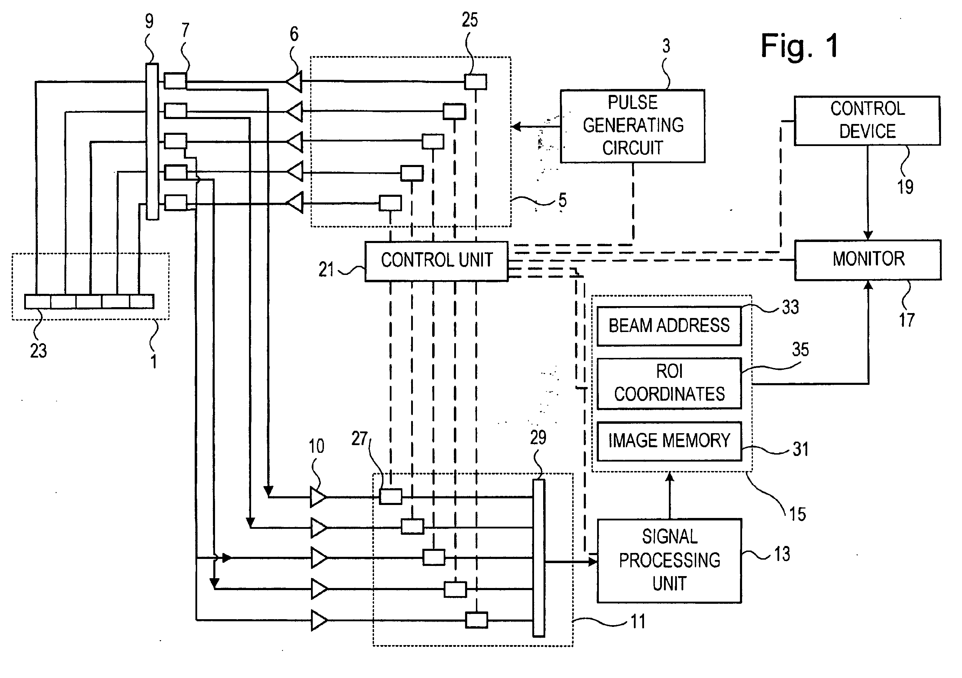

As shown in Fig.1, an ultrasound diagnostic apparatus according to the present embodiment includes: a probe 1; a pulse generating circuit 3; a transmitted wave focusing circuit 5; an amplifier 6; a transmission/reception separating circuit 7; a selector switch 9; an amplifier 10; a received wave phasing circuit 11; a signal processing unit 13; a memory 15; a monitor 17; a controller 19; and a control unit 21 including a computer, and the like.

The probe 1 may be one used in a known ultrasound diagnostic apparatus. For example, a plurality of transducers 23 for transmitting and receiving ultrasonic waves are arranged in a line as in an electronic sector type. The pulse generating circuit 3 is designed to generate an ultrasonic wave driving pulse (hereinafter referred to as a driving pulse) for driving each transducer 23.

The transmitted wave focusing circuit 5 is constructed to include a plurality of delay circuits 25 for adjusting supply timing of the driving pulses supplied to the transducers 23, and controls focal positions of ultrasound beams radiated from a plurality of the transducers 23 forming an aperture in accordance with instruction from the control unit 21. The driving pulses generated by the pulse generating circuit 3 are subjected to focusing processing with the transmitted wave focusing circuit 5, amplified by the amplifier 6, and supplied to the transmission/reception separating circuit 7. The driving pulses which have been supplied to the transmission/reception separating circuit 7 are supplied to the transducers 23 via the selector switch 9.

The plurality of the transducers 23 convert the supplied driving pulses into ultrasound signals, and radiate the ultrasound beams inside an object to be examined (not shown). The ultrasound beams radiated to the object are reflected inside the object to be reflected echo signals, and received by the respective transducers 23 forming the aperture. Each received signal received by the transducers 23 is supplied to the amplifier 10 via the selector switch 9 and the transmission/reception separating circuit 7, and is amplified therein. The received signals amplified by the amplifier 10 are supplied to the received wave phasing circuit 11.

The received wave phasing circuit 11 is a circuit for adjusting phases of the received signals received by the respective transducers 23 so as to convert them into signals with their received signals from a desired portion in the living body emphasized, which circuit includes a delay circuit 27 and an adder 29. The delay circuit 27 outputs the received signals supplied from the respective transducers 23 of the probe 1 after a predetermined time, and outputs them to the adder 29. The adder 29 adds up the received signals delay-controlled by the delay circuit 27 and outputs the resultant signal to the signal processing unit 13.

The signal processing unit 13 processes the received signals subjected to phasing with the received wave phasing circuit 11, generates image data of one or a plurality of ultrasound beams forming a tomogram, and stores the image data into the memory 15. The memory 15 includes an image memory 31 for storing received beam data sequentially generated by the signal processing unit 13 at every transmission and every reception of ultrasonic waves, a beam address memory 33 for storing a beam address, being an ultrasound beam identification code which is determined for each ultrasound beam, and an ROI coordinate memory 35 for storing coordinates of a preset ROI. The controller 19 is an input means for inputting commands of an operator, the controller formed by an interface such as a trackball or a mouse.

The control unit 21 is respectively connected to the pulse generating circuit 3, the transmitted wave focusing circuit 5, the received wave phasing circuit 11, the signal processing unit 13, the memory 15, the monitor 17, and the controller 19, to control upon instruction therefrom. According to the present embodiment, the transmitted wave focusing circuit 5 and the control unit 21 form a sound pressure adjusting means for adjusting a sound pressure of ultrasound beams in the ROls. The operator can set various diagnostic conditions by commanding the control unit 21 so from the controller 19.

Hereinafter, the operation and the detailed construction of the ultrasound diagnostic apparatus according to the present embodiment will be described. In the ultrasound diagnostic apparatus according to the present embodiment, for example, a portion including a lesion in a living body is pictured prior to the contrast medium imaging so as to obtain an image of a cross section of the lesion, i.e., a tomogram for setting ROIs, and it is displayed on the monitor 17. The ROIs are set in the diagnosis mode and on the above-described tomogram of the lesion displayed on the monitor 17. Next, a contrast medium is injected into a blood vessel and scanning is done while respective focal points of ultrasound beams are adjusted to be converged on the ROIs. By making uniform the effects of the contrast medium in the ROIs utilizing the contrast medium mode for making clear the image, a clear tomogram is obtained. The contrast medium is, for example, one known as generating bubbles in bloodstream. In this contrast medium, bubbles irradiated by ultrasound beams are broken and/or deformed. By collecting sound components generated when the bubbles are broken or deformed and imaging, a clear diagnostic image of portions where the contrast medium exists can be obtained.

An operator first pictures images of the lesion in the diagnostic mode. To this end, the probe 1 is applied to be in contact with the body surface of the object, or with the surface of an organ when abdominal surgery is being conducted, and is held so that the ultrasonic wave transmitting plane thereof is directed inside the living body. When the operator commands a start of picturing through the controller 19, the control unit 21 instructs the pulse generating circuit 3 and the transmitted wave focusing circuit 5 in response thereto. Thus the pulse generating circuit 3 and the transmitted wave focusing circuit 5 are activated to supply driving pulses amplified by the amplifier 6 to the respective transducers 23, and ultrasonic waves are emitted from the transducers 23 inside the object so that the wavefronts of the ultrasonic waves from the transducers 23 simultaneously arrive at a focal point set by the transmitted wave focusing circuit 5. That is, ultrasound beams are radiated inside the object. Here, the transmitted wave focusing circuit 5 performs the well-known transmission focusing processing. The drive timing of each transducer 23 is appropriately delayed by the transmitted wave focusing circuit 5. Therefore, the ultrasonic waves transmitted from the respective transducers 23 are synthesized and deflected to be ultrasound beams, and are focused at the focal point. In picturing a tomogram for setting ROIs in the diagnostic mode, a two-dimensional tomogram is obtained by scanning with the ultrasound beams while retaining a focal distance of each transmitted ultrasound beam at a predetermined depth set by the transmitted wave focusing circuit 5. In the scan with the ultrasound beams, scan lines in different directions are sequentially set by the control unit 21, and beam addresses are sequentially determined respectively in correspondence with the scan lines. The beam address is an identification code of ultrasound beam and is stored in the beam address memory 33.

The ultrasound beams sequentially transmitted in the scanning direction propagate inside the object. A part of the ultrasound beams is reflected when passing through a point where an acoustic impedance changes to be reflected echoes. The reflected echoes of the ultrasound beams reflected from the area where the ultrasound beams are radiated are received by the transducers 23. The transducers 23 convert the echoes into electrical signals in the order of reception and output the received signals. The received signals output from the transducers 23 are supplied to the received wave phasing circuit 11 via the selector switch 9, the transmission/reception separating circuit 7, and the amplifier 10. The received signal group input into the received wave phasing circuit 11 is subjected to phasing processing and is supplied to the signal processing unit 13 including a digital scan converter. The received beam signals input into the signal processing unit 13 are subjected to predetermined signal processing and are written in the image memory 31. Image signals acquired by performing ultrasound scan inside the object with plural times of ultrasound transmission and reception and written in the image memory 31 are read out when necessary, converted into coordinates of a display coordinate system, and displayed as an ultrasound image on the monitor 17. That is, the tomogram generated by the signal processing unit 13 is stored in the image memory 31, and the data of the tomogram stored in the image memory 31 are read out and displayed by the monitor 17. A well-known ultrasound diagnostic apparatus is applicable to these parts related to the diagnostic mode.

Meanwhile, when a plurality of ROIs such as lesions in the object exist and the depths thereof are respectively different, the contrast effects of contrast medium at the respective ROIs become inhomogeneous, and visibility of the tomogram obtained in the contrast medium mode is deteriorated in some cases. The ultrasound beams radiated inside the object are focused by the transmission focusing processing. Accordingly, the sound pressure of ultrasound beams is maximized in the vicinity of the focal point where the ultrasound beams are focused, and is decreased as the ultrasound beams move away from the focal point in proportion to a degree that the ultrasound beams are not focused.

In short, the sound pressure of ultrasound beams radiated inside the object gradually increases from the probe 1 to the focal depth, has its maximum value in the vicinity of the focal depth, and decreases in response to the depth deeper than the focal depth. The contrast medium is, for example, a solution mixed with bubbles in it, and a sound wave is generated due to crash and/or deformation of the bubbles. A characteristic sound wave is generated by the contrast medium when it receives an ultrasound beam. The characteristic sound wave becomes large when the sound pressure of transmitted ultrasound beams increases because the bubbles are burst and/or deformed.

As described above, because the behavior of the contrast medium largely varies depending on the sound pressure of the radiated ultrasound beams, if the sound pressures of the ultrasound beams radiated to the various portions of the object are different, the contrast degree is different even through the conditions of the portions are in similar conditions. Accordingly, when the depths of portions to be observed are different, the sound pressure of the ultrasound beams has a distribution that its peak is in the vicinity of the focal depth. Even if the conditions of ROIs are substantially the same and the concentration of bubbles in the contrast medium is substantially uniform, the contrast degrees of the contrast medium are different to deteriorate visibility. In a portion in the vicinity of the focal depth where the sound pressure of the ultrasound beams is large, crash and/or deformation of bubbles of the contrast medium are apt to occur, whereby the intensity of signals received from the contrast medium becomes high and the contrast degree brought about by the contrast medium is improved.

On the contrary, in a portion distant from the focal depth where the sound pressure of the ultrasound beams is small, crash and/or deformation of bubbles hardly occur and thus a strong sound wave is not generated, whereby the intensity of signals received from the contrast medium is weak and the contrast degree of the contrast medium is deteriorated. Thus, in a tomogram obtained by a conventional ultrasound diagnostic apparatus in which ultrasound beams in one scan are radiated with a constant focal distance, an ROI in the vicinity of the focal depth is emphasized in an image display due to the difference of the contrast degree of the contrast medium. Accordingly, sufficiently uniform contrast effects are not obtained in ROIs of various depths, and it is difficult to judge whether the reason for the difference in the contrast comes from the difference in conditions of the respective ROIs or from the difference in the sound pressures, whereby the clinical evaluation becomes difficult.

Therefore, in an ultrasound diagnostic apparatus according to an embodiment of the present invention, portions to be observed are set as ROIs, and the focal distances of ultrasound beams respectively radiated to the ROIs are modified during a scan. That is, the focal distances of the ultrasound beams for scanning the ROIs are variably controlled.

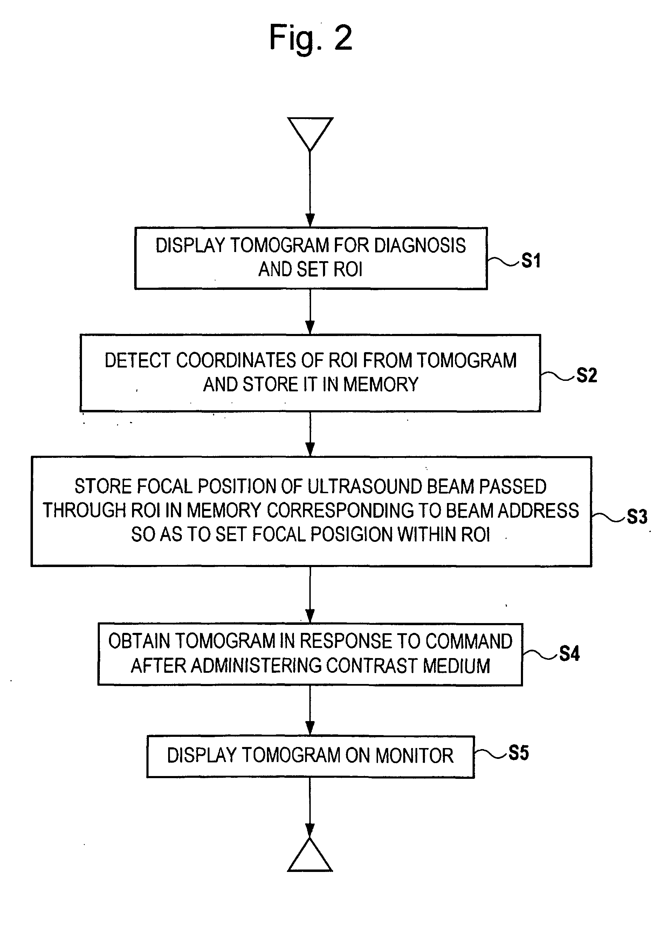

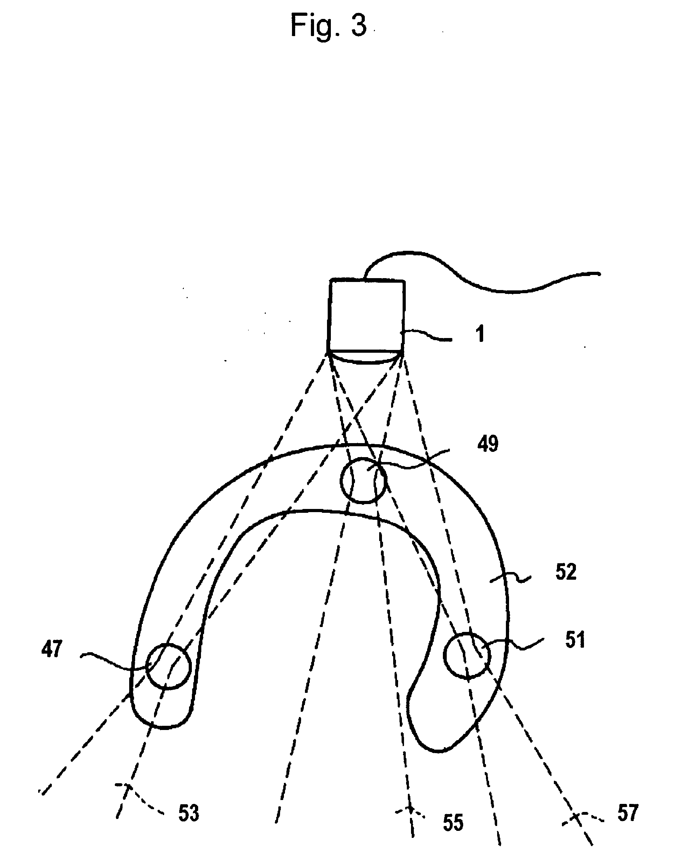

Here, the imaging procedure in the operation of the contrast medium mode in the ultrasound diagnostic apparatus according to this embodiment will be described with reference to Fig.2 and Fig.3. In the contrast medium mode, as shown in Fig.2, the apparatus is set to an ROI setting mode by operating the controller 19, and a plurality of ROIs 47, 49, and 51 shown in Fig.3 are set in a tomogram for setting the diagnostic ROI displayed on the monitor 17 in Step S1. In Fig.3, marks O represent ROIs 47, 49, and 51 set on a cardiac muscle 52, and broken lines represent ultrasound beams 53, 55, and 57 passing through the ROIs 47, 49, and 51. In step S1 and step S2, the control unit 21 reads out coordinates of the ROIs 47, 49, and 51, input them on the tomogram and stores them into the memory 15. In step S2 and step S3, in order to set focal points of the ultrasound beams 53, 55, and 57 passing through the respective ROIs 47, 49, and 51, the control unit 21 finds the focal points by calculation corresponding to beam addresses and stores them into the memory 15. In step S3 and step S4, after injecting the contrast medium, the tomogram is obtained in response to instructions of an operator. In step S4 and step S5, the obtained tomogram is displayed on the monitor 17. The imaging procedure is thus constructed.

In step S1, while the operator displays a tomogram on the monitor 17 in real time according to the diagnosis mode for setting ROIs, the ROIs 47, 49, and 51 are set by a controller 19 using an interface such as a mouse or a track ball, as shown in Fig.3. By setting these ROls 47, 49, and 51, for example ROI marks or the like indicating positions and ranges of the ROIs 47, 49, and 51 are displayed on the monitor 17, and superposed on a diagnostic image. The size of the ROI mark displayed may be appropriately changed depending on the size of a lesion.

In step S2, the above-set coordinates of the ROIs 47, 49, and 51, for example, the coordinates of predetermined positions of the ROIs 47, 49,and 51 are read out from the tomogram and are stored into an ROI coordinate memory 35 in correspondence with the image memory 31 and the beam address memory 33.

In step S3, the control unit 21 detects the respective beam addresses of the ultrasound beams 53, 55, and 57 passing through the above-set ROIs 47, 49, and 51 on the basis of, for example, the ROI coordinate memory 35 into which the coordinates of predetermined positions of the ROIs 47, 49, and 51 are stored. The focal point of the ultrasound beam 53 radiated from the probe 1 and passes through the ROI 47 is calculated so that it is located within the ROI 47, and the calculated focal point is stored in the beam address memory 33 in correspondence with its beam address. The ultrasound beam 55 passing through the ROI 49 and the ultrasound beam 57 passing through the ROI 51 are stored in the beam address memory in a similar manner. The focal distance of each of the ultrasound beams 53, 55, and 57 can be found by calculating the respective distance to the ROIs 47, 49, and 51 from a central transducer 23 being a reference point among a plurality of transducers 23 provided on the probe 1.

In step S4, when the contrast medium is administered to the object and the operator inputs instructions with the controller 19, the input by the controller 19 is transmitted to the control unit 21. This control unit 21 calculates time delay of driving pulses supplied to the respective transducers 23 on the basis of the focal points of the respective ultrasound beams stored in the beam address memory 33, and gives instructions to the pulse generating circuit 3 and the transmitted wave phasing circuit 5 so as to transmit ultrasound beams with the above-set focal depths. The transmitted wave focusing circuit 5 performs focusing processing on the respective ultrasound beams 53, 55, and 57 according to the instructions from the control unit 21 so that the ultrasound beams 53, 55, and 57 are focused in the ROIs 47, 49, and 51. The probe 1 sequentially transmits a plurality of ultrasound beams including the ultrasound beams 53, 55, and 57.

The term "focusing processing" is to narrow ultrasound beams and to reduce the sound field at the determined focal distance by shifting the operation timing of each transducer 23. For example, when the transducers 23 are arranged in a line, the operation timing of transducers 23 on the both ends is the priority, and those of the other transducers are gradually delayed toward the center of them. Further, by changing the delay time during one scan, the focal depths of the ultrasound beams 53, 55, and 57 can be changed. That is, the focal positions of the respective ultrasound beams sequentially transmitted are independently adjusted, and the ultrasound beams 53, 55, and 57 can be focused in the ROIs 47, 49, and 51, respectively.

The ultrasound beams transmitted to the object are reflected inside the object to generate reflected echoes, and as well break and/or deform the contrast medium due to the sound pressure so as to generate characteristic sound waves. The characteristic sound waves of the contrast medium and the reflected echoes are received by the transducers 23 and supplied to the received wave phasing circuit 11 via the selector switch 9 and the transmission/reception separating circuit 7, where they are subjected to phasing processing and output from the received wave phasing circuit 11. The received signals output by the received wave phasing circuit 11 are input into the signal processing unit 13, which generates a tomogram of the object on the basis of the series of received signals acquired during the scan, and the thus-generated tomogram is stored in an image memory 31 of the memory 15. Tomographic signals stored in the image memory 31 are subjected to scan-conversion into image signals and read out, and are displayed by the monitor 17 as an image display means. At this time, luminance of pixels is determined by the amplitude of the corresponding received beam signal.

Here, the received wave signal corresponding to each ultrasound beam represents time-series signal strength (amplitude) to the round-trip propagation time between transmission of ultrasound beam and reception of received beam signal, i.e., it represents a data row of amplitudes. Because the round-trip propagation time is in proportion to the depth from the probe 1 to a portion where the received beam signal is generated, a pixel location can be determined on the basis of the transmission direction of the ultrasound beam and the round-trip propagation time. Further, by determining the luminance of a pixel on the basis of amplitude of a received beam signal, a shading image can be generated. A tomogram displayed with light and shade based on the luminance of pixels is formed by scanning the object while changing a beam line of ultrasound beams, which image has, for example, a fan-shaped FOV. Processing of a received beam signal received by the transducers 23 can be performed in the same manner as in the diagnosis mode.

In step S5, the control unit 21 reads out the tomogram obtained and stored in step S4 from the image memory 31, and reads out the data of the coordinates of the ROIs 47, 49, and 51 from the ROI coordinate memory 35. These coordinates are linked to each other and displayed on a monitor 17 while superposing the ROI marks are superposed on the tomogram.

In this manner, the ultrasound diagnostic apparatus according to the present embodiment detects the coordinates of the set ROI 47, 49, and 51 and stores them in the memory 15; controls the focal positions of the ultrasound beams 53, 55, and 57 passing through the ROIs 47, 49, and 51, respectively during one scan used to construct one tomogram, on the basis of beam addresses of the ultrasound beams 53, 55, and 57 passing through the ROIs 47, 49, and 51; adjusts the focal positions of the ultrasound beams 53, 55, and 57 in the ROIs 47, 49, and 51, respectively; and adjusts the sound pressures of the ultrasound beams 53, 55, and 57 in the respective ROIs 47, 49, and 51 to be maximum regardless of the depth of the ROIs 47, 49, and 51, whereby the contrast degree of the contrast medium is improved and the visibility of a tomogram is thus improved.

Further, if an ultrasound beam is transmitted with a fixed focal distance, in an ROI distant from the focal position the ultrasound beam is not sufficiently focused, so that width of the ultrasound beam becomes large and the resolution is deteriorated. However, as in the ultrasound diagnostic apparatus according to the present embodiment, by controlling the focal distance of the ultrasound beams 53, 55, and 57 during scan so as to set the focal positions of the ultrasound beams 53, 55, and 57 passing through the ROIs 47, 49, and 51 within the respective ROIs 47, 49, and 51, the ultrasound beams 53, 55, 57 are sufficiently focused in the ROIs 47, 49, and 51, the width of the ultrasound beams 53, 55, and 57 becomes small, and thus, deterioration of resolution in the ROIs 47, 49, and 51 can be prevented. In addition, because the ROI marks are displayed on the monitor 17 indicating the ROIs 47, 49, and 51 and these ROI marks are superposed on the tomogram in the display, the operator can easily recognize positions of the ROIs 47, 49, and 51.

In the above embodiment, sound pressure adjusting means for equalizing sound pressure in ROIs by setting the focal positions of ultrasound beams in ROIs has been described. However, the present invention is not limited thereto, and the following modification is also possible.

By the control unit 21 controlling voltage of the driving pulse supplied to each of the transducers 23 according to the depth of each of the ROIs 47, 49 and 51 during one scan, the sound pressure herein is adjusted and the contrast effect of the contrast medium can be made uniform. In this case, a variable amplifier may be used as the amplifier 10, where the ROI of the maximum depth is set as the reference ROI and amplifications on other ROIs of shallower depth are lowered. Further, the sound pressure adjusting means may be structured by combining the above-described focal position adjustment of ultrasound beam and signal intensity adjustment of the driving pulse.

Further, the amplifier 6 is provided between the transmitted wave focusing circuit 5 and the transmission/reception separating circuit 7. However, it also may be included in the pulse generating circuit 3.

Further, according to the present embodiment, a tomogram of a cardiac muscle 52 in which ROIs sequentially exist is obtained. However, the ultrasound diagnostic apparatus according to the present invention may be applied not only to the diagnosis on the cardiac muscle 52 but also to imaging of a cross-section of another portion where ROIs are dispersed, such as HCC, i.e., hepatocellular carcinoma.

Further, as in the present embodiment, the ROI mark may be superposed on a tomogram displayed on the monitor 17. But alternatively, it is also desirable that a plurality of windows such as a window for displaying a diagnostic tomogram and a window for recognizing a location of ROI are provided on the monitor 17, and the ROI mark may be displayed superposing on the tomogram in the window showing the location of the ROI. Further, the form of the ROI mark may be a character, or the ROI mark may have any shape depending on the size of a lesion or the like. The point is that the ROI mark may have any shape as long as the location and the range of the ROI can be recognized so that a diagnostic image in which the contrast effect in the respective ROIs is made uniform can be obtained.

According to the present embodiment, one ultrasound beam passes through each of ROIs during one scan. However, for example, even when the area of the determined ROI is wide and a plurality of ultrasound beams pass through this ROI, in the same manner as in the ultrasound diagnostic apparatus according to the present embodiment, by controlling focal positions of the plurality of ultrasound beams passing through the ROI each of focal positions of the ultrasound beams can be set within the ROI.

In the ultrasound diagnostic apparatus according to the present embodiment, by setting the focal position of an ultrasound beam to be within an ROI and controlling voltage applied to the transducers 23, the sound pressure in each ROI is adjusted. However, the sound pressure in an ROI also can be adjusted by shifting the focal position of the ultrasound beam from the ROI. For example, the focal position of an ultrasound beam passing through the ROI at the deepest position is set to be within this ROI, and the shallower the other ROIs are, the further the focal position of the ultrasound beam passing through each ROI is removed from that ROI. In this manner, variation of sound pressure due to the degree of focus of ultrasound beams counterbalances variation of sound pressure due to the propagation and the attenuation of ultrasound beams through the object, whereby the sound pressure in each ROI can be made uniform.

Further, the setting of an ROI in the ultrasound diagnostic apparatus according to the present invention is not limited to the setting of an ROI in the present embodiment; for example, the apparatus itself may read in a tomogram and automatically search and set an ROI. Further, when a plurality of ROIs exist on one beam line, i.e., one beam line does not correspond to only one ROI, it is possible to transmit the same number of ultrasound beams as the plurality of ROIs on an identical beam line and control the focal position and voltage applied to each beam so as to make substantially uniform the sound pressure in each ROI.

Further, the focal position and a voltage applied of an ultrasound beam not passing through an ROI may be controlled to be a predetermined focal position and a predetermined voltage applied, respectively. It is also desirable to calculate paths which connect each ROI and control the focal position and the voltage applied so as to make uniform the sound pressure on this path.

According to the present embodiment, a plurality of discontinuous ROIs 47, 49, and 51 are set. However, it is also desirable to input and assign a straight line or a curved line connecting the plurality of ROIs into an ROI band, and control the focal positions of ultrasound beams and the voltage applied so as to make uniform the sound pressure of each ultrasound beam on this ROI band. The ROI band may be arbitrarily set by using a mouse, or a trackball, or the like. It also may be preset by the apparatus for each ROI. For example, if a blood vessel in which the contrast medium flows is set as a continuous ROI band, the contrast degree of the contrast medium in the blood vessel included in the ROI band is improved, and the time-sequential change of the contrast medium flowing in the blood vessel also can be observed.

Further, according to the present embodiment, the contrast medium is administered in step S4. However, the contrast medium may be administered prior to step S1, or in any one of steps S1 to S3. Furthermore, it is also possible to set an ROI having an inhomogeneous contrast effect after administering the contrast medium and obtain a tomogram thereof in the contrast medium mode. It is sufficient as long as, after administering the contrast medium, at least one of the focal position of the ultrasound beam radiated or the voltage applied to the transducers 23 or both is controlled to respectively adjust the sound pressure of the ultrasound beams radiated to the contrast medium in the ROIs, to thereby uniform the contrast effect.

## Claims

1. An ultrasound diagnostic apparatus which radiates ultrasound beams generated by driving a probe to an object to be examined into which a contrast medium is injected, generates a tomogram of the object on the basis of an ultrasound signal reflected from the object, and displays the image, comprising:
input means for setting on the tomogram a plurality of regions of interest (ROI) through which the contrast medium passes, and
sound pressure adjusting means for adjusting the ultrasound beams to substantially equalize the sound pressure to the contrast medium that passes through the plurality of ROIs set by the input means in the first ROI and in other ROIs.

2. An ultrasound diagnostic apparatus according to claim 1, wherein the plurality of the ROIs include an ROI band.

3. An ultrasound diagnostic apparatus according to claim 1, wherein the sound pressure adjusting means calculates distances from the probe to the ROIs, variably controls focal positions of the ultrasound beams according to the distance, and adjusts the sound pressures.

4. An ultrasound diagnostic apparatus according to claim 1, wherein the sound pressure adjusting means adjusts the sound pressure by controlling voltage of a driving pulse supplied to each transducer of the probe.

5. An ultrasound diagnostic apparatus according to claim 1, wherein the sound pressure adjusting means adjusts the sound pressure by varying a focal position of the ultrasound beam.

6. An ultrasound diagnostic apparatus according to claim 1, wherein the sound pressure adjusting means adjusts the sound pressure by controlling voltage of a driving pulse supplied to each transducer of the probe and varying the focal positions of the ultrasound beams.

7. An ultrasound diagnostic apparatus according to claim 1, wherein the sound pressure adjusting means reads out position coordinates of a plurality of the ROIs from the tomogram, stores them corresponding to the beam addresses, and controls the focuses of the ultrasound beams on the basis of the beam addresses so that the focuses of the ultrasound beams are located within the ROIs.

8. An ultrasound diagnostic apparatus according to claim 1 and claim 2, wherein the shape of a mark or the like given to the ROIs or the ROI band is varied according to the size of a lesion, and the mark or the like is displayed superposed on the tomogram.

9. An ultrasound diagnostic apparatus according to claim 1, wherein the sound pressure adjusting means calculates a path connecting a plurality of the ROIs and controls the ultrasound beams so as to make uniform the sound pressure along the path.

10. An ultrasound diagnostic apparatus according to claim 2, wherein the ROI band is determined by sequentially inputting and assigning a path connecting a plurality of ROIs with a straight line or a curved line.

11. An ultrasound diagnostic apparatus according to claim 1, wherein the sound pressure adjusting means maximizes the sound pressure in the ROIs.

12. An ultrasound diagnostic apparatus according to claim 1 and claim 2, wherein time-sequential change of a contrast medium flowing in the ROIs or in the ROI band is displayed in the image.

13. An ultrasound imaging method including:
a first step of radiating ultrasound beams generated by driving a probe to an object to be examined, generating a first tomogram of the object on the basis of an ultrasound signal reflected from the object, and displaying the image;
a second step of setting a plurality of ROIs from the first tomogram;
a third step of collecting information on the plurality of the ROIs from the first tomogram;
a fourth step of storing focal positions of the ultrasound beams in correspondence with the information of a plurality of the ROIs;
a fifth step of administering a contrast medium into the above-set ROIs;
a sixth step of transmitting ultrasound beams to a plurality of ROIs at the above-stored focal positions; and
a seventh step of displaying a second tomogram including the ROIs into which the contrast medium has been administered.

14. An ultrasound imaging method according to claim 13, wherein the sixth step includes a step of variably controlling the focal positions of the ultrasound beams in accordance with the distance from the probe to the ROIs.

15. An ultrasound imaging method according to claim 13, wherein the fourth step includes a step of reading out position coordinates of a plurality of the ROIs from the first tomogram and storing them in correspondence with beam addresses.

16. An ultrasound imaging method according to claim 15, wherein the sixth step includes a step of transmitting the ultrasound beams on the basis of the beam addresses.

17. An ultrasound imaging method according to claim 13, wherein the plurality of the ROIs includes an ROI band.

18. An ultrasound imaging method according to claim 17, wherein the ROI band is determined by sequentially inputting and assigning a path connecting the plurality of the ROIs with a straight line or a curved line.

19. An ultrasound imaging method according to claim 13, wherein the seventh step includes a step of displaying time-sequential change of the contrast medium flowing the ROIs.
